# EUROPÄISCHE PATENTSCHRIFT

(11) **EP 1 785 742 B1**
(45) Veröffentlichungstag und Bekanntmachung des Hinweises auf die Patenterteilung: **14.05.2008**
(21) Anmeldenummer: 05024671.9
(22) Anmeldetag: 11.11.2005
(51) Int. Cl.: G01S 7/52, G06T 7/00

(54) **Bestimmung von Schallgeschwindigkeitsfaktoren in Ultraschallbildern eines Körpers**
Determination of sound velocity in ultrasound images
Détermination de la vitesse du son dans des images échographiques

(43) Veröffentlichungstag der Anmeldung: 16.05.2007
(73) Patentinhaber: BrainLAB AG, 85551 Kirchheim/Heimstetten (DE)
(72) Erfinder: Haimerl, Martin, Dr., 82205 Gilching (DE)
(74) Vertreter: Schwabe - Sandmair - Marx

(56) Entgegenhaltungen:
- US-A1- 2005 080 333
- KRUCKER J F ET AL: "Sound speed estimation using ultrasound image registration" BIOMEDICAL IMAGING, 2002. PROCEEDINGS. 2002 IEEE INTERNATIONAL SYMPOSIUM ON JULY 7-10, 2002, PISCATAWAY, NJ, USA,IEEE, 7. Juli 2002 (2002-07-07), Seiten 437-440, XP010600619 ISBN: 0-7803-7584-X
- DUNCAN ET.AL.: "Self-calibrating Ultrasound-to-CT Bone Registration" MICCAI (CONF. PROCEEDINGS), LNCS 3749, 26. Oktober 2005 (2005-10-26), Seiten 605-612, XP009061078 Springer Verlag Heidelberg
- FOOKES C ET AL INSTITUTE OF ELECTRICAL AND ELECTRONICS ENGINEERS: "The use of mutual information for rigid medical image registration: a review" IEEE 2002 INTERNATIONAL CONFERENCE ON SYSTEMS, MAN AND CYBERNETICS. (SMC'02). YASMINE HAMMAMET, TUNESIA, OCT. 6 - 9, 2002, IEEE INTERNATIONAL CONFERENCE ON SYSTEMS, MAN, AND CYBERNETICS, NEW YORK, NY : IEEE, US, Bd. VOL. 7 OF 7, 6. Oktober 2002 (2002-10-06), Seiten 689-694, XP010623558 ISBN: 0-7803-7437-1
- KRÜCKER ET.AL.: "Registration-based sound speed estimation in phantoms with acoustically vaporized droplets" 2002 IEEE ULTRASONICS SYMPOSIUM, 2002, Seiten 1729-1732, XP009061154
- GLOVER ET AL: "Reconstruction of ultrasound propagation speed in soft tissue: time of flight tomography" IEEE TRANSACTIONS ON SONICS AND UTLRASONICS, Bd. SU-24, Nr. 4, 22. Juli 1977 (1977-07-22), Seiten 229-234, XP009061095
- LAVALLEE S ET AL: "Matching Of Medical Images For Computed And Robot Assisted Surgery" ENGINEERING IN MEDICINE AND BIOLOGY SOCIETY, 1991. VOL.13: 1991., PROCEEDINGS OF THE ANNUAL INTERNATIONAL CONFERENCE OF THE IEEE ORLANDO, FL, USA 31 OCT.-3 NOV. 1991, NEW YORK, NY, USA,IEEE, US, 31. Oktober 1991 (1991-10-31), Seiten 39-40, XP010101564 ISBN: 0-7803-0216-8
- SHIPLEY J A ET AL: "Automated quantitative volumetric breast ultrasound data-acquisition system" ULTRASOUND IN MEDICINE AND BIOLOGY, NEW YORK, NY, US, Bd. 31, Nr. 7, Juli 2005 (2005-07), Seiten 905-917, XP004944624 ISSN: 0301-5629

## Beschreibung

Die Erfindung betrifft die Bestimmung von Schallgeschwindigkeitsfaktoren in Ultraschallbildern eines Körpers. Insbesondere betrifft die Erfindung auch die Korrektur solcher Schallgeschwindigkeitsfaktoren.

Die Tatsache, dass die Schallgeschwindigkeit von dem Medium abhängig ist, durch das der Schall hindurch geht, kann bei der Ultraschall-Bilderfassung (z.B. bei der Bilderfassung für den menschlichen Körper) zu quantitativen Ungenauigkeiten führen. Dies bedeutet, dass die im Ultraschallbild wiedergegebenen Strukturen zwar der Form nach richtig dargestellt werden, jedoch die Entfernung vom Ultraschallkopf eventuell eine andere ist als auf dem Bild wiedergegeben. Im diagnostischen Bereich ist eine Korrektur der Schallgeschwindigkeitsfaktoren (= Einfluss unterschiedlicher Schallgeschwindigkeiten in unterschiedlichen durchdrungenen Medien) weniger wichtig, da auf diesem Feld die exakte bzw. absolute Position eines Objekts nicht von großer Wichtigkeit ist. Weil Ultraschallbilder aber meist zu diagnostischen Zwecken verwendet werden, sind bisher nur wenige Versuche gemacht worden, Schallgeschwindigkeitsfaktoren zu bestimmen bzw. zu korrigieren oder geometrische Verzerrungen und Abberationsfehler zu korrigieren. Es sind Versuche bekannt, Ultraschallbilder direkt zu registrieren, wobei lediglich die Ultraschallbilder selbst verwendet werden, beispielsweise zwei Schallbilder, in denen korrespondierende Strukturen gesucht werden. Jedoch leiden diese Versuche sehr unter den starken Störungen in den erfassten Bilddaten sowie an einer großen Anzahl von Artefakten, Problemen mit dem Sammeln von Daten aufgrund von Streueffekten und numerischen Schwierigkeiten.

Ein weiterer Ansatz zur Berücksichtigung bzw. Korrektur unterschiedlicher Schallgeschwindigkeitsfaktoren, der in Barratt et. al. "Self-Calibrating Ultrasound-to-CT Bone Registration" aus MICCAI (Conf. Proceedings), LNCS 3749, Springer Verlag, S. 605 bis 612 vorgeschlagen wurde, liegt in der zusätzlichen Optimierung von Skalierungsparametern während einer 3-dimensionalen Registrierung von Ultraschallbild-Datensätzen, z.B. auf einen CT-Datensatz. Bei dieser Methode werden die für die grundlegende Aufnahmegeometrie des Ultraschall-Bildgebungsprozesses maßgeblichen Parameter in das Registrierungsverfahren integriert. Da bei diesem Ansatz die Optimierung alle Ultraschall-Schichtaufnahmen gleichförmig behandelt, erfolgt eine globale Anpassung der Skalierungsparameter. Somit kann auch für die Schallgeschwindigkeitsfaktoren lediglich eine globale Schätzung bzw. Korrektur erfolgen.

Eine lokale Abschätzung von Schallgeschwindigkeitsfaktoren wird zum Teil in Verfahren zur Laufzeit-US-Tomographie (time-of-flight tomography) vorgenommen. Dies wurde z.B. in Glover, Sharp: Reconstruction of Ultrasound Propagation Speed Distributions in Soft Tissue: Time-of-Flight Tomography aus IEEE Transactions on Sonics and Ultrasonics, Vol. 24, S. 229 bis 234, 1977 durchgeführt. Jedoch sind aufgrund technischer Limitationen wie z.B. Rauschen, geometrische Verzerrungen, geringe Schalldurchdringung und weitere Artefakte sowie durch inhärente mathematische Eigenschaften, insbesondere durch die numerische Instabilität des für die Rekonstruktion der Schallgeschwindigkeitsfaktoren zu lösenden mathematischen Problems, diese Ansätze mit erheblichen Schwierigkeiten verbunden, die einen praktischen Einsatz der Verfahren für diagnostische Zwecke ebenso wie für die Unterstützung medizinischer Navigationsverfahren stark einschränkt. Die auf diese Weise ermittelten Daten sind für eine exakte Abschätzung von Schallgeschwindigkeitsfaktoren zu unzuverlässig.

Der Artikel von Krücker et al. mit dem Titel "Sound speed estimation using automatic ultrasound registration" aus IEEE Transactions on Ultrasonic, Ferroelectrics and Frequency Control, 51(9): 1095 bis 1106, 2004 (D1) beschreibt einen Versuch, bei welchem beispielsweise zwei aus verschiedenen Blickrichtungen aufgenommene Ultraschallbilder mit korrespondierenden Strukturen direkt registriert werden. Anhand der gewonnenen Daten wird letztendlich eine durchschnittliche Schallgeschwindigkeit durch das entsprechende Gewebe berechnet.

Das Dokument US 2005/0080333 A1 offenbart eine Vorrichtung, mit welcher die momentane Position eines medizinischen Instruments innerhalb eines behandelnden Körperteils bestimmt werden kann. Dazu wird zuerst das Körperteil mit Hilfe der MR-Methode vermessen und registriert. Anhand dieser Registrierung kann daraufhin ein sich innerhalb des zu behandelnden Körperteils befindliches medizinisches Instrument mit Hilfe von mehreren aus verschiedenen Blickrichtungen aufgenommenen Ultraschallbildern lokalisiert werden.

Es ist die Aufgabe der vorliegenden Erfindung, ein Verfahren bereit zu stellen, mit dem die Schallgeschwindigkeitsfaktoren in Ultraschallbildern eines Körpers zuverlässig bestimmt werden können. Insbesondere soll eine Korrektur dieser Faktoren ermöglicht werden.

Diese Aufgabe wird erfindungsgemäß durch ein Verfahren gemäß dem Anspruch 1 gelöst. Die Unteransprüche beschreiben vorteilhafte Ausführungsformen der vorliegenden Erfindung.

Erfindungsgemäß stellt die vorliegende Erfindung ein Verfahren zur Bestimmung von Schallgeschwindigkeitsfaktoren und Ultraschallbildern eines Körpers bereit, das die vorliegenden Schritte umfasst:
- ein Körper-Referenzmodell wird bereitgestellt,
- aus verschiedenen Richtungen werden mindestens zwei Ultraschallbilder einer inneren Körperregion erfasst,
- die Ultraschallbilder oder spezielle Sätze der Ultraschallbilder werden zweidimensional oder dreidimensional zu einem entsprechenden Bereich des Referenzmodells registriert,
- die Registrierung jedes Ultraschallbildes wird mit der Registrierung mindestens eines anderen Ultraschallbilds, das aus einer anderen Richtung erfasst wurde, verglichen,
- aus dem Vergleich von Referenzmodell-Punktabständen für eine bestimmte registrierte Struktur wird für jede einzelne Ultraschallbild-Richtung der Schallgeschwindigkeitsfaktor bestimmt.

Vorteilhafterweise lässt sich so eine robuste und zuverlässige Bestimmung bzw. auch Korrektur der Schallgeschwindigkeitsfaktoren bereitstellen, weil eine zuverlässige Referenz verwendet wird, nämlich das Referenzmodell. Dieses Referenzmodell kann vorab unter Verwendung eines bekannten, medizintechnischen Tracking- bzw. Navigationssystems in einem Raumkoordinatensystem registriert werden. Es stellt damit eine sichere Referenz und Abgleichungsmöglichkeit für die Ultraschallbilder dar. Wenn im vorliegenden Kontext der Begriff "Registrierung" verwendet wird, so ist damit eine Zuordnung von Strukturen oder Punkten auf Strukturen in unterschiedlichen Bilddatensätzen gemeint, also grundsätzlich eine Identifizierung gleicher Punkte, Linien oder Bereiche in unterschiedlichen Bilddatensätzen bzw. Bildern. Mit der Erfindung wird es nun nicht mehr notwendig, Ultraschallbilder aufeinander zu registrieren, was von Vorteil ist, wenn man bedenkt, dass die Bilder sehr viele Störungen aufweisen und nur eine Teilinformation (richtungsabhängig) über anatomische Strukturen bieten.

Erfindungsgemäß werden einzelne Ultraschall-Schichtaufnahmen oder Gruppen davon einzeln registriert (im Gegensatz zur globalen Registrierung, wie sie im oben beschriebenen Artikel von Barratt et al. vorgeschlagen wird). Aus der Kombination dieser individuellen Registrierungen oder einem Vergleich mit einer Referenzregistrierung ist für jede Sichtlinie eine Abschätzung der Schallgeschwindigkeit bzw. der Schallgeschwindigkeitsfaktoren möglich. Somit kann eine verständliche, detailliertere und robuste Schallgeschwindigkeitskorrektur durchgeführt werden. Es ist möglich, spezielle Strategien für die Akquirierung der Ultraschalldaten zu entwickeln, um die Abschätzung der Schallgeschwindigkeiten in relevanten Bereichen zu optimieren. Grundsätzlich könnte auch eine (teilweise) Ultraschalltomographie (time-of-flight tomography) mit diesem Ansatz durchgeführt werden, wobei auch hier der Bezug auf das Referenzmodell für eine zuverlässigere Abschätzung der Schallgeschwindigkeitsfaktoren sorgt.

Gemäß einer Ausführungsform der Erfindung weist das Körper-Referenzmodell einen Bilddatensatz aus einem bildgebenden, medizintechnischen Verfahren auf, insbesondere einen CT-Bilddatensatz, einen MR-Bilddatensatz, einen SPECT- oder PET-Bilddatensatz oder einen Röntgen-Projektionsbild-Datensatz.

Ferner kann das Körper-Referenzmodell einen Bilddatensatz aufweisen, der ein statistisches oder geometrisches Modell eines Körpers umfasst.

Die verschiedenen Ultraschall-Erfassungseinrichtungen können im Wesentlichen innerhalb einer Ebene liegen, und vorzugsweise einen Winkel von im Wesentlichen 20° bis 30° zueinander aufweisen. Es ist möglich, das Verfahren so auszugestalten, dass vor der zwei- oder dreidimensionalen Registrierung eine dreidimensionale grobe oder Vor-Registrierung für die Ultraschall- und Modellbilddatensätze erfolgt.

Je nach Ausführungsform kann der zu registrierende Bereich des Referenzmodells ein zweidimensionaler Bereich bzw. eine Ultraschall-Schichtaufnahme oder ein dreidimensionaler räumlicher Bereich sein.

Gemäß einer Ausführungsvariante wird eine zweidimensionale Registrierung jedes Ultraschallbildes mit der Registrierung nur eines oder einiger oder aller anderen Ultraschallbilder verglichen, wobei die Bilder aus einer anderen Richtung in im Wesentlichen derselben Ebene erfasst wurden.

Der Vergleich der Punktabstände für mehrere Ultraschallbilder oder Ultraschallbild-Sätze aus unterschiedlichen Richtungen kann gleichzeitig erfolgen. Es ist im Rahmen der Erfindung möglich, den Vergleich der Punktabstände für die Ultraschallbilder oder Ultraschallbild-Sätze mittels der Berechnung von Schnittpunkten zweier Ultraschall-Betrachtungslinien aus unterschiedlichen Richtungen durchzuführen, die auf denselben Punkt des Referenzmodells zielen.

Es besteht ferner die Möglichkeit, die Bestimmung der Schallgeschwindigkeitsfaktoren iterativ mit einer medizintechnischen, dreidimensionalen Registrierungsmethode durchzuführen.

Die Erfindung umfasst ferner ein Korrekturverfahren für den Schallgeschwindigkeitsfaktor in Ultraschallbildern, bei dem der Schallgeschwindigkeitsfaktor gemäß einem der Verfahren bestimmt wird, die oben beschrieben wurden, und dann kompensiert wird. Bei der Kompensation können lokale Schallgeschwindigkeitsunterschiede bzw. Schallgeschwindigkeitsfaktoren-Unterschiede eliminiert oder verringert werden, und es besteht außerdem die Möglichkeit, bei der Kompensation ein Relaxationskriterium in die Berechnung der Schallgeschwindigkeitsfaktoren zu integrieren.

Die Erfindung betrifft ferner ein Programm, das, wenn es auf einem Computer läuft oder in einem Computer geladen ist, den Computer veranlasst ein wie oben beschriebenes Verfahren durchzuführen. Sie betrifft ferner ein Computerprogramm-Speichermedium, das ein solches Programm aufweist.

Die Erfindung wird im Weiteren anhand einer Ausführungsform und mit Hilfe der beiliegenden Zeichnungen näher erläutert. Sie kann jedes hierin beschriebene Merkmal einzeln oder in jedweder Kombination aufweisen. In den Zeichnungen zeigen:
- Figur 1: einen Ultraschallkopf mit Navigationsreferenz, sowie dessen Bildbereich und Bildebene;
- Figur 2: verschiedene Bildbereiche eines Ultraschallkopfes in unterschiedlichen Ebenen an einem Knochen;
- Figur 3: die Registrierung einer Körperstruktur mit Hilfe eines Referenzmodells in schematischer Darstellung sowie anhand von Ultraschallbildern; und
- Figur 4: einen Vergleich unterschiedlicher Registrierungen durch Punktreferenzen.

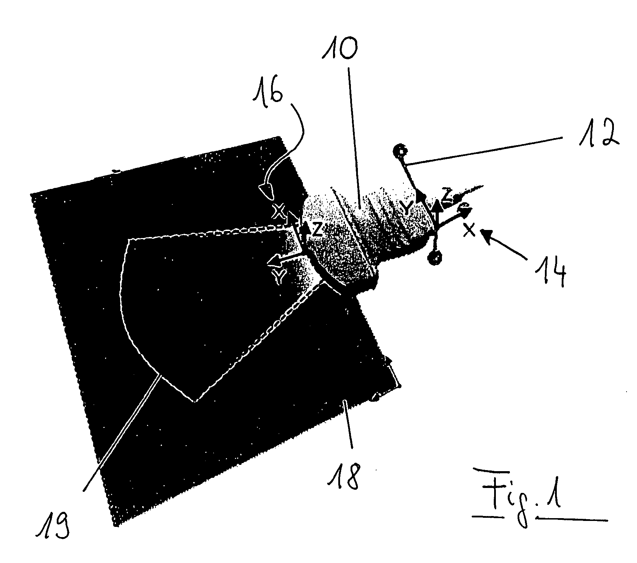

Ein Ultraschallkopf, wie er im Rahmen der vorliegenden Erfindung verwendet werden kann, ist in Figur 1 dargestellt. Der Kopf trägt das Bezugszeichen 10, und an dem Kopf 10 ist ein Referenzstern 12 befestigt. Mit Hilfe des Referenzsterns 12 kann der Kopf positionell geortet und verfolgt (getrackt) werden, und zwar mit Hilfe eines medizintechnischen Tracking- bzw. Navigationssystems. Dieses nicht dargestellte Navigationssystem verwendet ein erstes Koordinatensystem x,y,z, das mit dem Bezugszeichen 14 angedeutet ist.

Der Kopf 10 kann Bilder in einer Ebene 18 erfassen, und zwar im Ultraschall-Bildausschnitt 19, die im Weiteren auch als Ultraschall-Schichtaufnahme bezeichnet wird. Die Ultraschall-Schichtaufnahme 19 liegt in der Ebene 18, und der Kopf spannt grundsätzlich sein eigenes Koordinatensystem auf, das ebenfalls x,y,z genannt wird und mit dem Bezugszeichen 16 angedeutet ist. Bei einer festen Referenz 12 unterscheiden sich die Koordinatensystem 14 und 16 dem Grunde nach nicht.

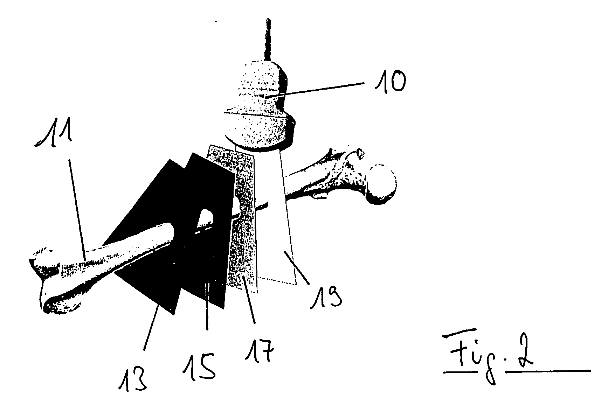

Die Figur 2 zeigt nochmals grundsätzlich die Ultraschall-Datenakquirierung und mit dem Kopf 10 werden im dargestellten Fall Ultraschallbilder eines Knochens 11 aufgenommen, nämlich Schichtaufnahmen 13, 15, 17, 19, die in unterschiedlichen Ebenen und in einem Winkel zueinander liegen.

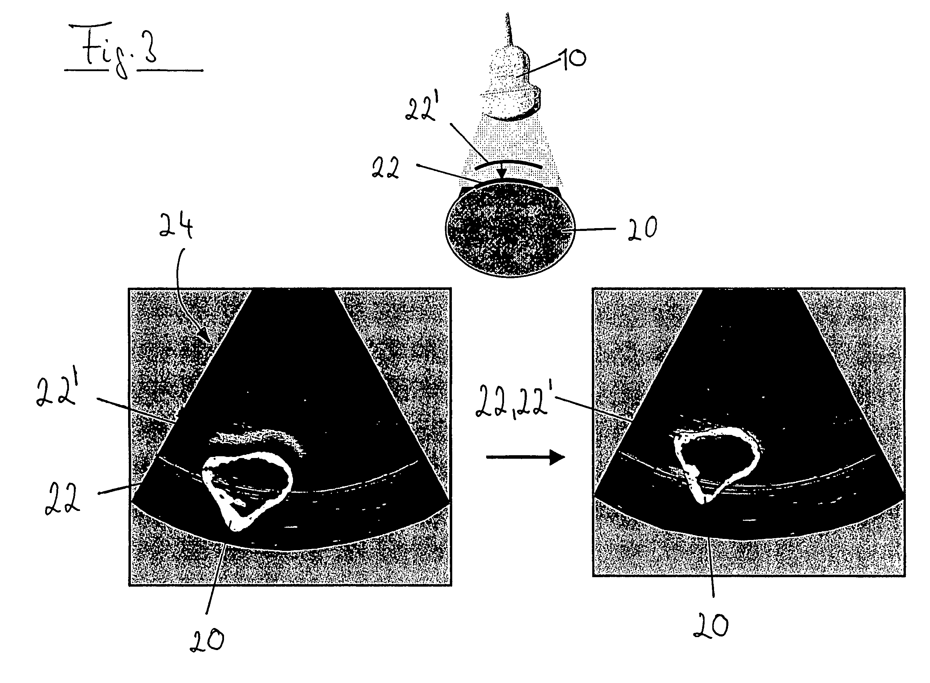

Im Ultraschallbild kann sich eine Struktur (beispielsweise ein mittlerer Abschnitt des Knochens 11) dann als gebogene Linie abbilden, und schematisch ist dies in der oberen Ansicht der Figur 3 nochmals dargestellt. Hier wird ganz allgemein eine Ultraschallaufnahme mit dem Kopf 10 von der Struktur 20 gemacht. Die Struktur 20 hat eine obere Kante 22. Dies ist die obere Kante, die auch im Ultraschallbild dargestellt werden kann.

Man kann sich die obere mittlere Darstellung der Figur 3 so vorstellen, dass die Struktur 20 die tatsächliche Lage wiedergibt, wie sie beispielsweise aus einem im Operationsraum registrierten Bilddatensatz hervorgeht (z.B. CT-Datensatz) oder mittels eines Navigationssystems im Operationsraum lokalisiert bzw. registriert worden ist. Der Ultraschallkopf 10 zeigt aber aufgrund von Schallgeschwindigkeitsfaktoren die Kante 22 der Struktur 20 nicht an ihrer tatsächlichen Stelle, sondern an einer anderen Stelle, nämlich an der mit 22' gezeigten Stelle.

Dieselbe Situation zeigt das linke untere Bild in Figur 3, in dem tatsächlich eine Ultraschallaufnahme, also eine Schichtaufnahme 24 zu sehen ist. In der Schichtaufnahme ist die Struktur 20 in ihrer tatsächlichen Lage, also mit der oberen Kante 22 an der tatsächlichen Raumposition eingeblendet, und die weiße Kante 22' zeigt, wo der Ultraschallkopf 10 diese Kante sieht. Dieser Unterschied in den überlagerten Bilddatensätzen gibt nun eine quantitative Aussage darüber ab, wie sehr die Schallgeschwindigkeitsfaktoren die Längenbestimmung im Ultraschallbild beeinflussen. Mit einer solchen quantitativen Aussage, also mit der Kenntnis der Auswirkung der Schallgeschwindigkeitsfaktoren lässt sich nun eine Kompensation durchführen. Nach der Kompensation wird der Ultraschallkopf die Strukturkante 22' genau dort abbilden, wo auch die Kante 22 des Referenzmodell-Datensatzes abgebildet wird. Man weiß nun für die vorliegende Situation zumindest für eine Betrachtungsrichtung, wie die Kompensation der Schallgeschwindigkeitsfaktoren durchgeführt werden muss, und man kann hiermit auch quantitativ aussagefähige Ultraschallbilder erstellen.

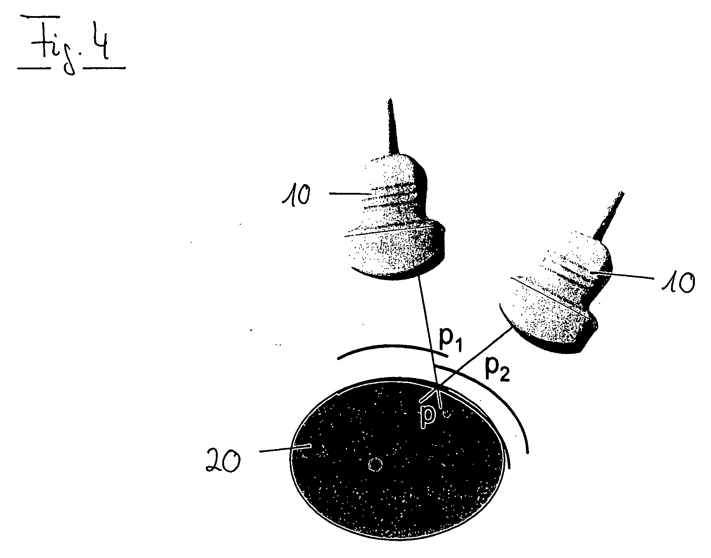

Die Figur 4 zeigt schließlich einen Vergleich unterschiedlicher Registrierungen mit Hilfe von Punktreferenzen bei Ultraschall-Schichtaufnahmen, die in annäherndderselben Ebene, aber winkelversetzt aufgenommen werden. Die Ultraschallbilder lassen sich in einer solchermaßen aufgespannten Ebene zweidimensional registrieren, wobei die vorher beschriebene Abstandserfassung punktweise durchgeführt werden kann. Die Figur 4, zeigt wie die Registrierung realiter mit dem Punkt P auf der Struktur 20 durchzuführen ist. Es ist schwierig, den Punkt P aus nur einer Richtung mit guter Genauigkeit zu erfassen, weil die Schallgeschwindigkeitsfaktoren sich in "Blickrichtung" des Ultraschallkopfes auswirken. Aus diesem Grund wird nicht nur eine Abbildung P1 des Punktes P erzeugt, sondern in derselben Ebene nur in versetztem Winkel (z.B. 20°- 30°) eine zweite Punktabbildung P2: Weil die beiden Ultraschall-Schichtaufnahmen mit den Abbildungen P1 und P2 in einem Winkel zueinander stehen, öffnet sich die Möglichkeit, den Lenkungsfehler zu korrigieren, den Punkt P eindeutig zu bestimmen und zu registrieren und auch den Abbildungsversatz genau zu erfassen. Mit dieser genauen Erfassung lässt sich dann eine ebenso genaue Kompensation einleiten.

## Patentansprüche

1. Verfahren zur Bestimmung von Schallgeschwindigkeitsfaktoren in Ultraschallbildern eines Körpers, mit den folgenden Schritten:
- ein Körper-Referenzmodell wird bereitgestellt,
- aus verschiedenen Richtungen werden mindestens zwei Ultraschallbilder einer inneren Körperregion erfasst,
- die Ultraschallbilder oder spezielle Sätze der Ultraschallbilder werden zweidimensional oder dreidimensional zu einem entsprechenden Bereich des Referenzmodells registriert,
- die Registrierung jedes Ultraschallbildes wird mit der Registrierung mindestens eines anderen Ultraschallbilds, das aus einer anderen Richtung erfasst wurde verglichen,
- aus dem Vergleich von Referenzmodell-Punktabständen für eine bestimmte registrierte Struktur wird für jede einzelne Ultraschallbild-Richtung der Schallgeschwindigkeitsfaktor bestimmt.

2. Verfahren nach Anspruch 1, bei dem das Körper-Referenzmodell einen Bilddatensatz aus einem bildgebenden medizintechnischen Verfahren aufweist, insbesondere ein CT-Bilddatensatz, ein MR-Bilddatensatz, ein SPECT- oder PET-Bildatensatz oder ein Röntgen-Projektionsbilder-Datensatz.

3. Verfahren nach Anspruch 1 oder 2, bei dem das Körper-Referenzmodell einen Bilddatensatz aufweist, der ein statistisches oder geometrisches Modell des Körpers umfasst.

4. Verfahren nach einem der Ansprüche 1 bis 3, bei dem die verschiedenen Ultraschallbild-Erfassungsrichtungen im Wesentlichen innerhalb einer Ebene liegen und vorzugsweise einen Winkel von im Wesentlichen 20 bis 30° zueinander aufweisen,

5. Verfahren nach einem der Ansprüche 1 bis 4, bei dem vor der zwei- oder dreidimensionalen Registrierung eine dreidimensionale grobe oder Vorregistrierung für die Ultraschall- und Modellbilddatensätze erfolgt.

6. Verfahren nach einem der Ansprüche 1 bis 5, bei dem der zu registrierende Bereich des Referenzmodells ein zweidimensionaler Bereich bzw. eine Ultraschall-Schichtaufnahme oder ein dreidimensionaler räumlicher Bereich ist.

7. Verfahren nach einem der Ansprüche 1 bis 6, bei dem die zweidimensionale Registrierung jedes Ultraschallbildes mit der Registrierung nur eines oder einiger oder aller anderen Ultraschallbilder verglichen wird, wobei die Bilder aus einer anderen Richtung zum Teil in im Wesentlichen derselben Ebene erfasst wurden.

8. Verfahren nach einem der Ansprüche 1 bis 7, bei dem der Vergleich der Registrierungen für mehrere Ultraschallbilder oder Ultraschallbild-Sätze aus unterschiedlichen Richtungen gleichzeitig erfolgt.

9. Verfahren nach einem der Ansprüche 1 bis 8, bei dem der Vergleich der Punktabstände für die Ultraschallbilder oder Ultraschallbild-Sätze durchgeführt wird mittels der Berechnung von Schnittpunkten zweier Ultraschall-Bertrachtungslinien aus unterschiedlichen Richtungen, die auf denselben Punkt des Referenzmodells zielen.

10. Verfahren nach einem der Ansprüche 1 bis 9, bei dem die Bestimmung der Schallgeschwindigkeitfaktoren iterativ mit einer medizintechnischen dreidimensionalen Registrierungsmethode erfolgt.

11. Korrekturverfahren für den Schallgeschwindigkeitsfaktor in Ultraschallbildern, bei dem der Schallgeschwindigkeitsfaktor gemäß einem der vorhergehenden Ansprüche bestimmt und dann kompensiert wird.

12. Verfahren nach Anspruch 11, bei dem bei der Kompensation lokale Schallgeschwindigkeitsunterschiede bzw. Schallgeschwindigkeitsfaktoren-Unterschiede eliminiert oder verringert werden.

13. Verfahren nach Anspruch 11 oder 12, bei dem bei der Kompensation ein Relaxationskriterium in die Berechnung der Schallgeschwindigkeitsfaktoren integriert wird.

14. Programm, das, wenn es auf einem Computer läuft oder in einem Computer geladen ist, den Computer veranlasst, ein Verfahren gemäß einem der Ansprüche 1 bis 13 durchzuführen.

15. Computerprogramm-Speichermedium, das ein Programm nach Anspruch 14 aufweist.

## Claims

1. A method for determining speed-of-sound factors in ultrasound images of a body, comprising the steps of:
- providing a body reference model;
- capturing at least two ultrasound images of an internal body region from different directions;
- registering the ultrasound images or specific sets of ultrasound images, two-dimensionally or three-dimensionally, with respect to a corresponding region in the reference model;
- comparing the registration of each ultrasound image with the registration of at least one other ultrasound image captured from a different direction;
- determining the speed-of-sound factor for each individual ultrasound image direction from the comparison of reference model point distances for a particular registered structure.

2. The method according to claim 1, wherein the body reference model comprises an image data set from a medical imaging method, in particular a CT image data set, an MR image data set, a SPECT or PET image data set or an x-ray projection image data set.

3. The method according to claim 1 or 2, wherein the body reference model comprises an image data set comprising a statistical or geometric model of the body.

4. The method according to any one of claims 1 to 3, wherein the various ultrasound capturing means lie substantially within one plane and preferably exhibit an angle of substantially 20° to 30° to each other.

5. The method according to any one of claims 1 to 4, wherein the ultrasound and model image data sets are roughly or preliminarily registered three-dimensionally, before they are two-dimensionally or three-dimensionally registered.

6. The method according to any one of claims 1 to 5, wherein the region of the reference model to be registered is a two-dimensional region or ultrasound tomograph or a three-dimensional spatial region.

7. The method according to any one of claims 1 to 6, wherein the two-dimensional registration of each ultrasound image is compared with the registration of just one or a few or all of the other ultrasound images, wherein the images from a different direction have to some extent been captured in substantially the same plane.

8. The method according to any one of claims 1 to 7, wherein the registrations for a number of ultrasound images or sets of ultrasound images from different directions are compared simultaneously.

9. The method according to any one of claims 1 to 8, wherein the point distances for the ultrasound images or sets of ultrasound images are compared by calculating intersection points of two ultrasound lines of view from different directions which point to the same point in the reference model.

10. The method according to any one of claims 1 to 9, wherein the speed-of-sound factors are determined iteratively, using a medical three-dimensional registration method.

11. A correcting method for the speed-of-sound factor in ultrasound images, wherein the speed-of-sound factor is determined in accordance with any one of the preceding claims, and is then compensated for.

12. The method according to claim 11, wherein compensating eliminates or reduces local differences in the speed-of-sound or speed-of-sound factor.

13. The method according to claim 11 or 12, wherein in compensating, a relaxation criterion is integrated into calculating the speed-of-sound factors.

14. A program which, when it is running on a computer or is loaded onto a computer, causes the computer to perform a method in accordance with any one of claims 1 to 13.

15. A computer program storage medium comprising a program according to claim 14.

## Revendications

1. Procédé de détermination de facteurs de vitesse du son dans des images échographiques du corps, qui présente les étapes suivantes :
- préparation d'un modèle de référence du corps,
- saisie d'au moins deux images échographiques d'une partie intérieure du corps dans des directions différentes,
- mise en correspondance bidimensionnelle ou tridimensionnelle des images échographiques ou des ensembles spéciaux d'images échographiques avec une partie correspondante du modèle de référence,
- comparaison entre la mise en correspondance de chaque image échographique et la mise en correspondance d'au moins une autre image échographique qui a été saisie dans une autre direction et
- à partir de la comparaison entre les distances entre les points du modèle de référence pour une structure donnée mise en correspondance, détermination du facteur de vitesse du son dans chaque direction des images échographiques.

2. Procédé selon la revendication 1, dans lequel le modèle de référence du corps présente un jeu de données d'image qui provient d'un procédé technique d'imagerie médicale, en particulier un jeu de données d'image de tomographie assistée par ordinateur (CT), un jeu de données d'image de résonance magnétique (MR), un jeu de données d'image de tomographie calculée d'émission de photons simples (SPECT) ou de tomographie par émission de protons (PET) ou un jeu de données d'images projetées aux rayons X.

3. Procédé selon les revendications 1 ou 2, dans lequel le modèle de référence du corps présente un jeu de données d'image qui comprend un modèle statistique ou géométrique du corps.

4. Procédé selon l'une des revendications 1 à 3, dans lequel les différentes directions de saisie des images échographiques sont essentiellement situées à l'intérieur d'un plan et forment entre elles un angle de préférence compris essentiellement entre 20 et 30°.

5. Procédé selon l'une des revendications 1 à 4, dans lequel on effectue une mise en correspondance tridimensionnelle grossière ou préalable des jeux de données d'images échographiques et d'images du modèle avant la mise en correspondance bidimensionnelle ou tridimensionnelle.

6. Procédé selon l'une des revendications 1 à 5, dans lequel la partie du modèle de référence qui doit être mise en correspondance est une partie bidimensionnelle ou un enregistrement tomographique par échographie ou une partie en trois dimensions.

7. Procédé selon l'une des revendications 1 à 6, dans lequel la mise en correspondance bidimensionnelle de chaque image échographique est comparée à la mise en correspondance d'une seule, de certaines ou de toutes les autres images échographiques, certaines images ayant été saisies dans une autre direction essentiellement dans le même plan.

8. Procédé selon l'une des revendications 1 à 7, dans lequel on compare simultanément les mises en correspondance de plusieurs images échographiques ou de plusieurs ensembles d'images enregistrées dans différentes directions.

9. Procédé selon l'une des revendications 1 à 8, dans lequel la comparaison entre les distances entre les points des images échographiques ou des ensembles d'images échographiques est réalisée par le calcul des points d'intersection de deux lignes d'observation des ultrasons dans différentes directions, qui visent le même point du modèle de référence.

10. Procédé selon l'une des revendications 1 à 9, dans lequel la détermination des facteurs de vitesse du son s'effectue par itération à l'aide d'une méthode de mise en correspondance tridimensionnelle de la technique médicale.

11. Procédé de correction du facteur de vitesse du son dans des images échographiques, dans lequel le facteur de vitesse du son est défini selon l'une des revendications précédentes et ensuite compensé.

12. Procédé selon la revendication 11, dans lequel la compensation élimine ou diminue des différences locales de vitesse du son ou des différences locales du facteur de vitesse du son.

13. Procédé selon les revendications 11 ou 12, dans lequel, lors de la compensation, un critère de relaxation est intégré dans le calcul de facteurs des vitesses du son.

14. Programme qui, lorsqu'il est exécuté sur un ordinateur ou est chargé dans un ordinateur, permet à l'ordinateur d'exécuter un procédé selon l'une des revendications 1 à 13.

15. Support de mémoire pour programme informatique, qui présente un programme selon la revendication 14.
